Europäisches Patentamt

European Patent Office

Office européen des brevets

(11) Numéro de publication : **0 477 112 B1**

(12) **FASCICULE DE BREVET EUROPEEN**

(45) Date de publication du fascicule du brevet :
25.05.94 Bulletin 94/21

(51) Int. Cl.⁵ : **C07C 55/14, C07C 51/14**

(21) Numéro de dépôt : **91420324.5**

(22) Date de dépôt : **13.09.91**

(54) **Procédé de préparation de l'acide adipique par hydrocarboxylation d'acides penteniques.**

(30) Priorité : **18.09.90 FR 9011714**

(43) Date de publication de la demande :
**25.03.92 Bulletin 92/13**

(45) Mention de la délivrance du brevet :
**25.05.94 Bulletin 94/21**

(84) Etats contractants désignés :
**BE DE ES FR GB IT NL**

(56) Documents cités :
**EP-A- 0 274 076**
**FR-A- 2 015 735**

(73) Titulaire : **RHONE-POULENC CHIMIE**
**25, quai Paul Doumer**
**F-92408 Courbevoie Cédex (FR)**

(72) Inventeur : **Denis, Philippe**
**16, Allée des Troenes**
**F-69150 Décines (FR)**
Inventeur : **Grosselin, Denis**
**12, Allée des Erables**
**F-69340 Francheville (FR)**

(74) Mandataire : **Vignally, Noel et al**
**Rhône-Poulenc Chimie**
**Direction de la Propriété Industrielle**
**Centre de Recherches des Carrières**
**B.P. 62**
**F-69192 Saint-Fons Cédex (FR)**

**Description**

La présente invention concerne un procédé de préparation de l'acide adipique par hydrocarboxylation d'acides penténiques, c'est-à-dire par réaction de l'eau et de l'oxyde de carbone sur au moins un acide penténique, en présence d'un catalyseur à base de rhodium et d'au moins un promoteur iodé.

Dans la demande de brevet européen n° 188 209 il est proposé un procédé de préparation d'acides dicarboxyliques linéaires, en particulier de l'acide adipique par réaction d'acides monocarboxyliques insaturés, en particulier l'acide pentène-3 oïque, de l'oxyde de carbone et d'eau en présence d'un catalyseur à base de rhodium et d'un promoteur iodé, la réaction étant conduite dans un solvant tel le chlorure de méthylène à une température de 100 à 240°C et sous une pression totale comprise entre 14 et 240 atm ; une température comprise entre 150 et 180°C et une pression totale comprise entre 24 et 40 atmosphères sont estimées préférables. La pression partielle du monoxyde de carbone est habituellement comprise entre 10 et 35 atm et de préférence entre 10 et 17 atm. Le choix du solvant est indiqué être critique dans le cadre du procédé en cause et il est même estimé que des solvants tels l'acide acétique sont indésirables en raison des faibles taux de linéarité obtenus en leur présence.

De même, il est affirmé que des solvants non polaires tels le cyclohexane et le toluène sont eux aussi indésirables en raison de leur propension à promouvoir directement la formation de produits branchés et indirectement celle des acides monocarboxyliques saturés.

Dans la demande de brevet européen n° 0 274 076, il est proposé un procédé de préparation d'acides carboxyliques linéaires par hydroxycarboxylation d'esters insaturés ou d'alcènes à insaturation terminale comportant de 4 à 16 atomes de carbone en présence d'un catalyseur à base de rhodium et d'un promoteur iodé. La réaction est conduite dans un solvant choisi indifféremment parmi le chlorure de méthylène, le dichloro-1,2 éthane et les solvants aromatiques et, un acide aliphatique ou aromatique de pKa compris entre 4,2 et 5,2 est présent comme accélérateur de la réaction. La pression partielle de l'oxyde de carbone est comprise entre 10 et 200 et, de préférence entre 13 et 20 atm.

Toutefois au départ d'esters penténiques on observe essentiellement la formation de monoadipate de méthyle.

Il a maintenant été trouvé qu'il est possible d'obtenir sélectivement l'acide adipique par hydrocarboxylation d'acides penténiques en présence d'un catalyseur à base de rhodium et d'un promoteur iodé dans un solvant choisi parmi les acides carboxyliques.

La présente invention a donc pour objet un procédé de préparation de l'acide adipique par réaction de l'eau et de l'oxyde de carbone sur au moins un acide penténique, en présence d'un catalyseur à base de rhodium et d'au moins un promoteur iodé, à une température comprise entre 100 et 240°C, sous une pression supérieure à la pression atmosphérique caractérisé en ce que

a) la réaction est mise en oeuvre dans un acide carboxylique aliphatique saturé ou aromatique, comportant au maximum 20 atomes de carbone et en ce que

b) la pression partielle de l'oxyde de carbone mesurée à 25°C est inférieure ou égale à 20 bar.

Par acide penténique, on entend dans le cadre de la présente invention l'acide pentène-2 oïque, l'acide pentène-3 oïque, l'acide pentène-4 oïque et leurs mélanges.

L'acide pentène-4 oïque conduit à de bons résultats, mais reste peu disponible.

L'acide pentène-3 oïque pris isolément ou en mélange avec ses isomères est plus particulièrement approprié compte-tenu de son accessibilité et des résultats satisfaisants auxquels il conduit dans le cadre du présent procédé.

Le procédé selon la présente invention nécessite la présence d'un catalyseur à base de rhodium. N'importe quelle source de rhodium est susceptible d'être mise en oeuvre.

A titre d'exemples de sources de rhodium susceptibles de convenir à la mise en oeuvre du présent procédé on peut citer :

Rh métal ; $Rh_2O_3$ ;

$RhCl_3$ ; $RhCl_3, 3H_2O$ ;

$RhBr_3$ ; $RhBr_3, 3H_2O$ ;

$RhI_3$ ; $Rh(NO_3)_3$ ; $Rh(NO_3)_3, 2H_2O$ ;

$Rh_2(CO)_4Cl_2$ ; $Rh_2(CO)_4Br_2$ ; $Rh_2(CO)_4I_2$ ;

$Rh(CO)Cl[P(C_6H_5)_3]_2$

$Rh[P(C_6H_5)_3]_2(CO)I$

$Rh[P(C_6H_5)_3]_3Br$

$Rh_4(CO)_{12}$ ; $Rh_6(CO)_{16}$ ; $Rh(CO)_2$ (acac) ;

$Rh(Cod)(acac)_2$ ; $Rh(acac)_3$ ;

$Rh_2(Cod)_2Cl_2 . Rh_2(CO_2CH_3)_4$ ;

$HRh(CO)[P(C_6H_5)_3]_3$ ;

(Cod = cyclooctadiène-1,5 ; acac : acétylacétonate)

Conviennent plus particulièrement à la mise en oeuvre du présent procédé :

$HRh(CO)[P(C_6H_5)_3]_3$ ;

$Rh(CO)Cl[P(C_6H_5)_3]_2$ ;

$Rh_2(Cod)_2Cl_2$ ;

$Rh_2(CO)_4Cl_2$ ;

$RhI_3$ ; $RhCl_3, 3H_2O$ ; $Rh(acac)_3$ ;

$Rh(Cod)(acac)_2$ ; $Rh_2(CO_2CH_3)_4$ ; $Rh_4(CO)_{12}$ ;

et $Rh_6(CO)_{16}$.

La quantité de rhodium à mettre en oeuvre peut varier dans de larges limites.

En général, une quantité exprimée en mole de rhodium métallique par litre de milieu réactionnel comprise entre $10^{-3}$ et $10^{-1}$ conduit à des résultats satisfaisants. Des quantités inférieures peuvent être mise en oeuvre ; on observe toutefois que la vitesse de réaction est faible. Des quantités supérieures n'ont d'inconvénients qu'au plan économique.

De préférence, la concentration de rhodium est comprise entre $5.10^{-3}$ et $10^{-2}$ (inclus) mol/l.

Par promoteur iodé on entend dans le cadre du présent procédé HI et les composés organoiodés capables de générer HI dans les conditions réactionnelles et, en particulier les iodures d'alkyles en $C_1$-$C_{10}$, l'iodure de méthyle étant plus particulièrement préconisé.

La quantité de promoteur iodé à mettre en oeuvre est en général telle que le rapport molaire I/Rh soit supérieur ou égal à 0,1. Il n'est pas souhaitable que ce rapport excède 20. De préférence, le rapport molaire I/Rh est compris entre 1 et 4 (inclus).

La présence d'eau est indispensable à la conduite du procédé selon la présente invention. En général, la quantité d'eau à mettre en oeuvre est telle que le rapport molaire eau/acide(s) penténique(s) soit compris entre 1 et 10 (inclus).

Une quantité inférieure présente l'inconvénient de limiter la conversion. Une quantité supérieure n'est pas souhaitable en raison de la perte d'activité catalytique observée.

Selon l'une des caractéristiques essentielles de la présente invention, la réaction est mise en oeuvre dans un acide carboxylique aliphatique saturé ou aromatique, comportant au maximum 20 atomes de carbone.

La nature précise de l'acide carboxylique n'est pas critique dans le cadre du présent procédé, pour autant que cet acide soit à l'état liquide dans les conditions réactionnelles.

A titre d'exemples d'acides carboxyliques susceptibles de convenir à la mise en oeuvre du procédé selon l'invention, on peut citer :

l'acide acétique, l'acide propionique, l'acide butyrique, l'acide valérique, l'acide adipique, l'acide benzoïque et l'acide phénylacétique.

De préférence on recourt à un acide carboxylique aliphatique en $C_1$-$C_4$. L'acide acétique convient plus particulièrement à la mise en oeuvre de la présente invention.

La quantité d'acide carboxylique présente dans le milieu réactionnel peut varier dans de larges limites, par exemple de 10 à 99 % (inclus) en volume du milieu réactionnel. De préférence cette quantité est comprise entre 30 et 90 % en volume (inclus).

Selon une autre caractéristique essentielle du présent procédé, la pression partielle de l'oxyde de carbone, mesurée à 25°C, est inférieure ou égale à 20 bar.

Lorsque la pression partielle de l'oxyde de carbone, mesurée à 25°C, est supérieure à cette valeur la sélectivité en diacides linéaire et/ou branchés est très faible et on note la formation de quantités importantes de méthyl-4-butyrolactone, composé indésirable.

Un minimum de pression partielle de l'oxyde de carbone de 0,5 bar (mesurée à 25°C) est préconisé.

De préférence, la pression partielle de l'oxyde de carbone, mesurée à 25°C, est inférieure ou égale à 10 bar.

On peut utiliser de l'oxyde de carbone sensiblement pur ou de qualité technique, tel qu'il se présente dans le commerce.

Comme indiqué en tête du présent mémoire, la température de réaction est comprise entre 100 et 240°C. Pour une bonne mise en oeuvre de la présente invention la température est comprise entre 160 et 190°C (inclus).

La réaction est conduite sous pression supérieure à la pression atmosphérique et, généralement, en phase liquide.

La pression totale peut varier dans certaines limites qui dépendront du mode opératoire retenu, de la pression partielle de l'oxyde de carbone et de celles des constituants du milieu réactionnel à la température réactionnelle choisie et, le cas échéant de la pression autogène du (ou des) acide(s) penténique(s) présents.

Le milieu réactionnel renferme l'acide carboxylique aliphatique saturé ou aromatique, de l'eau, une ou des sources de rhodium, un ou des promoteurs iodés et, le cas échéant tout ou partie du ou des acides penténiques engagés et des produits de la réaction.

En fin de réaction ou du temps imparti à celle-ci, on sépare l'acide adipique par tout moyen approprié par exemple par cristallisation et/ou distillation de l'acide carboxylique.

Les exemples ci-après illustrent la présente invention.

Exemple 1 :

Dans un autoclave en acier inoxydable (HASTELLOY B2) de 125 cm3 préalablement purgé à l'argon, on introduit :

325 mg (1,32 mmol) de rhodium sous forme de [RhCl(COD)]$_2$
0,64 g (4,5 mmol) de CH$_3$I
2 g (110 mmol) d'eau
5 g ( 50 mmol) d'acide pentène-3 oïque
50 cm$^3$ d'acide acétique

L'autoclave est fermé hermétiquement, placé dans un four agité et raccordé à l'alimentation de gaz sous pression. On admet à froid 2 bar de CO et on chauffe 175°C en 20 minutes. Lorsque cette température est atteinte, on régule la pression à 8 bar.

Après une durée de réaction de 20 minutes, l'absorption de CO a cessé ; l'autoclave est alors refroidi et dégazé.

La solution réactionnelle est analysée par chromatographie en phase gazeuse et par chromatographie liquide haute performance.

Les quantités de produits formés (rendement molaire par rapport à l'acide pentène-3 oïque chargé) sont les suivantes :

acide valérique        (Pa) :  ⌁COOH  =  1  %

acide pentène-4 oïque (P4) :  ⌁COOH  =  2,2 %

acide pentène-3 oïque (P3) :  ⌁COOH  =  13  %

acide pentène-2 oïque (P2) :  ⌁COOH  =  8,8 %

méthyl-4 butyrolactone (M4L) :  ⌁=O  =  8  %

acide éthylsuccinique  (A3) :  HOOC⌁COOH  =  3 %

acide méthylglutarique (A2) :  HOOC⌁COOH  =  23 %

acide adipique         (A1) :  HOOC⌁COOH  =  54 %

Le taux de linéarité (L) est de 67 %
Le taux de transformation de l'acide pentène-3 oïque (TT) est de 87 %.

Exemple 2 ; Essais Témoins (a) à (c) :

Dans l'autoclave et selon le mode opératoire, décrits pour l'exemple 1, on réalise une première série d'essais, en ne modifiant que la pression partielle de l'oxyde de carbone. Cette pression (à 175°C) est égale à la différence entre la pression totale ($P_T$) et la pression autogène de l'ordre de 4 bar.

Les conditions particulières ainsi que les résultats obtenus toutes conditions égales par ailleurs sont rassemblés dans le tableau (I) ci-après, dans lequel les conventions utilisées sont les mêmes que pour l'exemple 1 et t représente la durée de réaction en température.

## TABLEAU I

| Réf. | PT bar | t mn | TT % | Al % | L % | M4L % |
|------|--------|------|------|------|------|-------|
| 1 | 8 | 20 | 87 | 54 | 67 | 8 |
| 2 | 15 | 10 | 100 | 35 | 42 | 10 |
| a | 28 | 20 | " | 22 | 27 | 16 |
| b | 60 | 15 | " | 18 | 24 | 21 |
| c | 100 | 20 | " | 19 | 27 | 27 |

Ces résultats mettent en évidence le rôle déterminant d'une faible pression partielle de CO sur le taux de linéarité (L) et sur la quantité de lactone formée (M4L).

Exemples 3 à 5 ; essais témoins (d) à (f) :

Dans l'autoclave et selon le mode opératoire, décrits ci-avant on réalise une seconde série d'essais analogue à la précédente à ceci près que la charge contient 50 mmol d'acide pentène-4 oïque au lieu de l'acide pentène-3 oïque.

Les conditions particulières ainsi que les résultats obtenus, toutes conditions égales par ailleurs, figurent au tableau II ci-après :

## TABLEAU II

| Réf. | PT bar | t mn | TT % | Al % | L % | M4L % |
|------|--------|------|------|------|------|-------|
| 3 | 8 | 20 | 100 | 72 | 78 | 7 |
| 4 | 12 | 10 | " | 65 | 73 | 10 |
| 5 | 15 | 20 | " | 42 | 51 | 15 |
| d | 28 | 15 | " | 48 | 59 | 17 |
| e | 50 | 20 | " | 43 | 53 | 17 |
| f | 100 | 20 | " | 40 | 56 | 29 |

Exemple 6 ; essais témoins (g) et (h) :

Dans l'autoclave et selon le mode opératoire, décrits ci-avant on réalise une troisième série d'essais analogue à la première série à ceci près que la charge contient 50 mmol d'acide pentène-4 oïque au lieu de l'acide pentène-3 oïque et une quantité d'iodure de méthyle divisée par deux.

Les conditions particulières ainsi que les résultats obtenus, toutes conditions égales par ailleurs, figurent au tableau III ci-après :

## TABLEAU III

| Réf. | PT bar | t mn | TT % | Al % | L % | M4L % |
|------|--------|------|------|------|-----|-------|
| 6 | 8 | 20 | 100 | 71 | 80 | 8 |
| g | 50 | 20 | " | 43 | 60 | 26 |
| h | 100 | 35 | " | 36 | 58 | 36 |

Exemples 7 et 8 essais témoins (i) et (j) :

Dans l'autoclave et selon le mode opératoire, décrits pour les exemples 1 et 2, on réalise une quatrième série d'essais en utilisant 50 mmol d'acide pentène-2 oïque au lieu de l'acide pentène-3 oïque.

Les conditions particulières ainsi que les résultats obtenus, toutes conditions égales par ailleurs, figurent au tableau IV ci-après :

## TABLEAU IV

| Réf. | PT bar | t mn | TT % | Al % | L % | M4L % |
|------|--------|------|------|------|-----|-------|
| 7 | 8 | 40 | 35 | 23,5 | 55 | 4 |
| 8 | 12 | 90 | 77 | 27 | 49 | 7 |
| i | 28 | 100 | 98 | 17 | 30 | 6,5 |
| j | 50 | 120 | 97 | 16 | 28 | 7 |

Dans cette série d'essais on note la présence de quantité importantes ($\simeq$ 35-45 %) d'acide valérique ; dans l'ensemble des séries précédentes cet acide saturé, lorsqu'il est présent, ne représente que $\simeq$ 0,5 à 2 %.

Exemple 9 :

Dans l'autoclave et selon le mode opératoire, décrits précédemment on réalise un essais sur une charge constituée par :
- 25 mmol d'acide pentène-3 oïque
- 110 mmol d'eau
- 1,32 mmol de rhodium sous forme de [RhCl(COD)]$_2$
- 2,6 mmol d'iodure de méthyle et

EP 0 477 112 B1

- 50 cm3 d'acide acétique

Les résultats obtenus après 30 minutes de réaction à 175°C sous une pression totale de 8 bar sont les suivants :

- TT = 100 %
- A1 = 58 %
- L = 67 %
- M4L = 6 %

Exemple 10 :

On reproduit l'exemple 9 ci-avant à ceci près que la charge renferme 100 mmol d'acide pentène-3 oïque.
Les résultats obtenus après 80 minutes de réaction toutes conditions égales par ailleurs sont les suivants :

. TT = 99 %
. A1 = 58 %
. L = 68 %
. M4L = 6 %

Exemples 11 à 15 :

Dans l'autoclave et selon le mode opératoire, décrits précédemment, on réalise une cinquième série d'essais sur une charge renfermant :

- 1,32 mmol de rhodium sous forme de $[RhCl(COD)]_2$
- 2,5 mmol de $CH_3I$
- 110 mmol d'eau
- 50 mmol d'acide pentène-3 oïque
- 50 cm3 d'acide acétique

Les conditions particulières ainsi que les résultats obtenus figurent dans le tableau V ci-après :

## TABLEAU V

| Réf. | T °C | PT bar | P(CO) bar | t mn | TT % | A1 % | L % | M4L % |
|------|------|--------|-----------|------|------|------|-----|-------|
| 11 | 145 | 8 | 5 | 70 | 100 | 34 | 37 | 5 |
| 12 | 160 | 8 | 4 | 30 | " | 49 | 54 | 5 |
| 13 | 175 | 8 | 3 | 40 | " | 62 | 71 | 6,5 |
| 14 | 190 | 9 | 3 | 20 | 97 | 62 | 76 | 9,5 |
| 15 | 210 | 16 | 6 | 120 | 97 | 35 | 59 | 28 |

Dans l'exemple 11, on observe avant absorption une période d'induction de l'ordre de 40 mn.
Dans l'exemple 12 on observe avant absorption une période d'induction de l'ordre de 20 mn.

Exemples 16 à 23 :

On réalise une sixième série d'essais dans les conditions de l'exemple 13 en faisant varier la quantité de $[RhCl(COD)]_2$ et/ou celle de $CH_3I$ chargée.
Les conditions particulières ainsi que les résultats obtenus figurent dans le tableau VI ci-après :

7

(* Dans l'exemple 18 la quantité d'eau chargée n'est que de 10 mmol).

TABLEAU VI

| Réf. | Rh mmol | CH3I mmol | I/Rh | t mn | TT % | A1 % | L % | M4L % |
|---|---|---|---|---|---|---|---|---|
| 16 | 2,6 | 4,5 | 2 | 40 | 99 | 65 | 78 | 5,6 |
| 17 | " | 2,2 | 1 | 60 | 100 | 65 | 77 | 9,5 |
| 1 | 1,32 | 4,5 | 3,5 | 20 | 87 | 46,5 | 67 | 7 |
| 18* | " | " | " | 120 | 37 | 48 | 68 | 12 |
| 13 | " | 2,25 | 2 | 40 | 100 | 62 | 71 | 6,5 |
| 19 | " | 1,30 | 1 | 180 | 97 | 57 | 73 | 11,5 |
| 20 | " | 0,6 | 0,5 | (24h) | 80 | 32 | 78 | 29,5 |
| 21 | 0,65 | 1,3 | 2 | 90 | 100 | 55,5 | 65 | 8 |
| 22 | 0,33 | 2,45 | 7,4 | 60 | 99 | 38 | 52 | 17 |
| 23 | 0,33 | 0,6 | 2 | 210 | 88 | 43 | 59 | 8 |

**Revendications**

1. Procédé de préparation de l'acide adipique par réaction de l'eau et de l'oxyde de carbone sur au moins un acide penténique, en présence d'un catalyseur à base de rhodium et d'au moins un promoteur iodé, à une température comprise entre 100 et 240°C, sous une pression supérieure à la pression atmosphérique caractérisé en ce que

   a) la réaction est mise en oeuvre dans un acide carboxylique aliphatique saturé ou aromatique, comportant au maximum 20 atomes de carbone et en ce que
   b) la pression partielle de l'oxyde de carbone mesurée à 25°C est inférieure ou égale à 20 bar.

2. Procédé selon la revendication 1, caractérisé en ce que l'acide carboxylique est l'acide acétique.

3. Procédé selon la revendication 1 ou 2, caractérisé en ce que l'acide carboxylique représente au minimum 10 % en volume du milieu réactionnel liquide.

4. Procédé selon la revendication 3, caractérisé en ce que l'acide carboxylique représente de 30 à 90 (inclus) % en volume du milieu réactionnel liquide.

5. Procédé selon l'une quelconque des revendications précédentes, caractérisé en ce que la concentration en rhodium dans le milieu réactionnel est comprise entre $10^{-3}$ et $10^{-1}$ mol/l (inclus).

6. Procédé selon l'une quelconque des revendications précédentes, caractérisé en ce que le rapport molaire I/Rh est supérieur ou égal à 0,1.

7. Procédé selon l'une quelconque des revendications précédentes caractérisé en ce que le rapport molaire I/Rh est inférieur ou égal a 20.

8

8. Procédé selon l'une quelconque des revendications précédentes, caractérisé en ce que le rapport molaire eau/acide(s) penténique(s) est compris entre 1 et 10 (inclus).

9. Procédé selon l'une quelconque des revendications précédentes, caractérisé en ce que la température de réaction est comprise entre 160 et 190°C.

10. Procédé selon l'une quelconque des revendications précédentes, caractérisé en ce que la pression partielle de l'oxyde de carbone, mesurée à 25°C, est inférieure ou égale à 10 bar.

**Patentansprüche**

1. Verfahren zur Herstellung von Adipinsäure durch Umsetzung von Wasser und Kohlenoxid mit mindestens einer Pentensäure in Gegenwart eines Katalysators auf Rhodium-Basis und mindestens eines iodierten Promotors bei einer Temperatur zwischen 100 und 240°C eingeschlossen unter einem Druck oberhalb von Atmosphärendruck, dadurch gekennzeichnet, daß
   a) die Reaktion in einer gesättigten aliphatischen oder aromatischen Carbonsäure, die maximal 20 Kohlenstoffatome umfaßt, durchgeführt wird, und daß
   b) der Partialdruck des Kohlenoxids, gemessen bei 25°C, unterhalb von oder gleich 20 bar liegt.

2. Verfahren nach Anspruch 1, dadurch gekennzeichnet, daß die Carbonsäure Essigsäure ist.

3. Verfahren nach Anspruch 1 oder 2, dadurch gekennzeichnet, daß die Carbonsäure mindestens 10 Volumen-% des flüssigen Reaktionsmediums ausmacht.

4. Verfahren nach Anspruch 3, dadurch gekennnzeichnet, daß die Carbonsäure 30 bis 90 (eingeschlossen) Volumen-% des flüssigen Reaktionsmediums ausmacht.

5. Verfahren nach irgendeinem der vorangehenden Ansprüche, dadurch gekennzeichnet, daß die Rhodiumkonzentration im Reaktionsmedium zwischen $10^{-3}$ und $10^{-1}$ Mol/l (eingeschlossen) liegt.

6. Verfahren nach irgendeinem der vorangehenden Ansprüche, dadurch gekennzeichnet, daß das Molverhältnis I/Rh oberhalb von oder gleich 0,1 ist.

7. Verfahren nach irgendeinem der vorangehenden Ansprüche, dadurch gekennzeichnet, daß das Molverhältnis I/Rh unterhalb von oder gleich 20 ist.

8. Verfahren nach irgendeinem der vorangehenden Ansprüche, dadurch gekennzeichnet, daß das Molverhältnis Wasser/Pentensäure(n) zwischen 1 und 10 (eingeschlossen) liegt.

9. Verfahren nach irgendeinem der vorangehenden Ansprüche, dadurch gekennzeichnet, daß die Reaktionstemperatur zwischen 160 und 190°C eingeschlossen liegt.

10. Verfahren nach irgendeinem der vorangehenden Ansprüche, dadurch gekennzeichnet, daß der Partialdruck des Kohlenoxids, gemessen bei 25°C, unterhalb von oder gleich 10 bar ist.

**Claims**

1. Process for the preparation of adipic acid by reaction of water and carbon monoxide with at least one pentenic acid in the presence of a rhodium-based catalyst and at least one iodinated promoter, at a temperature between 100 and 240°C, under a pressure greater than atmospheric pressure, characterized in that
   a) the reaction is carried out in a saturated aliphatic or an aromatic carboxylic acid containing a maximum of 20 carbon atoms, and in that
   b) the partial pressure of carbon monoxide measured at 25°C is less than or equal to 20 bar.

2. Process according to claim 1 characterized in that the carboxylic acid is acetic acid.

3. Process according to claim 1 or 2 characterized in that the carboxylic acid represents at least 10 % by volume of the liquid reaction mixture.

4.  Process according to claim 3 characterized in that the carboxylic acid represents from 30 to 90 % (inclusive) by volume of the liquid reaction mixture.

5.  Process according to any one of the preceding claims, characterized in that the concentration of rhodium in the reaction mixture is between $10^{-3}$ and $10^{-1}$ mol/l (inclusive).

6.  Process according to any one of the preceding claims, characterized in that the I/Rh molar ratio is greater than or equal to 0.1.

7.  Process according to any one of the preceding claims, characterized in that the I/Rh molar ratio is less than or equal to 20.

8.  Process according to any one of the preceding claims, characterized in that the water/pentenic acid(s) molar ratio is between 1 and 10 (inclusive).

9.  Process according to any one of the preceding claims, characterized in that the reaction temperature is between 160 and 190°C.

10. Process according to any one of the preceding claims characterized in that the partial pressure of carbon monoxide measured at 25°C is less than or equal to 10 bar.